# EUROPEAN PATENT SPECIFICATION

(11) **EP 3 829 538 B1**
(45) Date of publication and mention of the grant of the patent: **02.07.2025**
(21) Application number: 18758670.6
(22) Date of filing: 31.07.2018
(51) Int. Cl.: A61K 9/107, A61K 9/06, A61K 47/14, A61K 47/32, A61K 47/44, A61K 31/69, A61P 17/00, A61P 17/06

(54) **TOPICAL COMPOSITION**
TOPISCHE ZUSAMMENSETZUNG
COMPOSITION TOPIQUE

(43) Date of publication of application: 09.06.2021
(73) Proprietor: MC2 Therapeutics Limited, Guildford, Surrey GU2 8XG (GB)
(72) Inventor: CRUTCHLEY, Nigel, Guildford GU2 8XG (GB)
(74) Representative: Boult Wade Tennant LLP
(86) International application number: PCT/GB2018/052191
(87) International publication number: WO 2020/025910

(56) References cited:
- WO-A1-2008/110826
- WO-A1-2017/093857
- WO-A1-2017/102565
- WO-A1-2018/013655
- WO-A1-2018/142117
- WO-A2-2007/095638
- US-A1- 2016 318 955
- EMILY IP ET AL: "P2712 Formulation, skin penetration, and anti-inflammatory activity of AN2728: A novel borinic acid ester", JOURNAL OF THE AMERICAN ACADEMY OF DERMATOL, MOSBY, INC, US, vol. 56, no. 2, 1 February 2007 (2007-02-01), pages AB177, XP005937376, ISSN: 0190-9622
- KARL BEUTNER ET AL: "P3327 AN2728 demonstrates significant efficacy in three phase Ib psoriasis microplaque trials", JOURNAL OF THE AMERICAN ACADEMY OF DERMATOLOGY, MOSBY, INC, US, vol. 60, no. 3, 1 March 2009 (2009-03-01), pages AB168, XP025964545, ISSN: 0190-9622, [retrieved on 20090224]
- EMILY IP ET AL: "P2756 Preclinical toxicology of AN2728, a novel borinic acid ester with anti-inflammatory activity", JOURNAL OF THE AMERICAN ACADEMY OF DERMATOL, MOSBY, INC, US, vol. 56, no. 2, 1 February 2007 (2007-02-01), pages AB188, XP005937420, ISSN: 0190-9622

## Description

The present invention relates to a topical composition. In particular, the invention relates to a topical composition comprising crisaborole having improved skin permeation, stability and/or patient compliance compared to existing crisaborole formulations.

Atopic dermatitis is a common, chronically relapsing, inflammatory skin disease. The exact cause of the disease is a matter of debate but it is characterised by eczematous lesions, dry skin and intense pruritus (itching). There is also strong evidence that the prevalence of atopic dermatitis has been increasing over recent years. The condition can vary from mild to severe with subsequent detriment to quality of life.

Current treatment programs include the use of emollient creams and then supplementing this with other therapies on a graduated scale. Topical application of a mild corticosteroid such as hydrocortisone acetate is usually the next step, with increasingly potent corticosteroids being utilised only if necessary. There are, however, a number of potential drawbacks associated with topical corticosteroids. These drawbacks, which apply especially to the more potent corticosteroids, can include skin thinning, tachyphylaxis and rebound phenomena. Due to these and other potential side effects, corticosteroids are not advised for use on the facial areas. This is despite the fact that atopic dermatitis that develops on the face can be the most detrimental to a patient's quality of life. Furthermore, corticosteroids are not recommended for continuous use, even though atopic dermatitis is a chronic disease.

Recently, crisaborole has been approved by the US Food and Drug Administration (FDA) for the treatment of mild to moderate atopic dermatitis in patients of at least two years of age. Crisaborole is a non-steroidal topical phosphodiesterase-4 (PDE-4) inhibitor. While its mechanism of action is not yet fully understood, it is believed that crisaborole inhibits PDE-4 in target cells. This, in turn, is thought to reduce the production of pro-inflammatory cytokines thought to cause the signs and symptoms of atopic dermatitis. Crisaborole is also being developed for other inflammatory dermatological conditions such as psoriasis.

The FDA-approved crisaborole formulation (trade name Eucrisa ^{®}) is a non-aqueous topical ointment having a crisaborole level of 2%. The absence of water limits the chances of chemical degradation due to hydrolysis or pH incompatibility and the occlusive nature of the bulk of the excipients creates a high degree of occlusion aiding permeation of the active. However, like most ointments, the lack of water and the presence of paraffin and wax components give the formulations a poor aesthetic profile (S.E. Wolverton, Comprehensive Dermatologic Drug Therapy 3rd Edition (2012), p13). This can potentially limit patient compliance.

US 2016/0318955 A1 discloses boron-containing small molecules as anti-inflammatory agents, and exemplifies crisaborole. The document further suggests that the boron compounds disclosed therein can be formulated into creams, and provides examples of cream formulations containing some boron-containing small molecules but not crisaborole itself. The cream formulations are all oil-in-water dispersions in which the oil phase is solidified by the inclusion of a solid emulsifying and structuring agent (8 wt% glyceryl monostearate in Examples 28B and 29). The solid emulsifier needs to be heated to be incorporated into the oil phase, but then solidifies the oil phase upon cooling post-emulsification. In WO 2017/093857 A1 these two examples are repeated but with crisaborole as the active. Emily IP et al, Journal of the American Academy of Dermatol, Mosby, Inc, US, vol. 56, no.2 (2007), p AB177, relates to the formulation, skin penetration and anti-inflammatory activity of a novel borinic acid ester AN2728. WO 2007/095638 A2 relates to boron-containing small molecules as anti-inflammatory agents. WO 2018/142117 A1 relates to a topical composition comprising a polyaphron dispersion.

Creams are advantageous over non-aqueous ointments in the sense that they generally have a better aesthetic profile and elicit improved patient compliance. However, US 2016/0318955 A1 makes no mention of the physical/chemical stability of the creams or whether the drug remains in solution. It is believed that crisaborole would have a tendency to crystallise out of the cream formulations disclosed in US 2016/0318955 A1, particularly when present at a relatively high concentration (e.g. 2%). This could, in turn, give rise to physical instability of the formulation. Poor solvation of the crisaborole may also have a negative effect on skin permeation.

Accordingly, it is one object of the present invention to provide a formulation that can deliver crisaborole into the skin with better aesthetics than prior art ointment formulations. In other words, it is one object of the present invention to provide a crisaborole formulation having better patient compliance than prior art ointment formulations.

It is an alternative and/or additional object to provide a crisaborole formulation having better skin penetration than prior art cream and/or ointment formulations.

It is an alternative and/or additional object to provide a cream formulation in which the crisaborole is fully solvated or at least more solvated than in existing cream formulations.

It is an alternative and/or additional object to provide a cream formulation having improved chemical and/or physical stability in comparison with existing cream formulations.

According to a first aspect, the present invention provides a composition for topical application according to claim 1.

Advantageously, the present inventors have found that the present invention, which includes a discontinuous liquid oil phase, achieves improved skin penetration compared with the formulations containing a solidified oil phase disclosed in US 2016/0318955 A1. The present inventors' finding that the use of a liquid oil phase improves skin penetration of crisaborole is surprising and unexpected, since this effect is not known for similar alternative pharmaceutically active ingredients. Without wishing to be bound by theory, it is believed that while the solid emulsifying and structuring agent serves to physically stabilise the composition, its incorporation at the levels disclosed in US 2016/0318955 A1 inhibits permeation of the active. In particular, it is believed that the solidification of the oil phase caused by the solid emulsifying and structuring agent restricts the ability of the crisaborole to diffuse into the skin. Indeed, it seems that the diffusion of crisaborole is unusually inhibited by a solidified oil phase.

The present inventors have also found that the use of a discontinuous liquid oil phase, rather than the solid oil phase disclosed in US 2016/0318955 A1, does not compromise the physical stability of the composition. This is surprising given that a solid phase would be expected to physically stabilise the formulation.

The present invention will now be described further. In the following passages different aspects/embodiments of the invention are defined in more detail. Each aspect/embodiment so defined may be combined with any other aspect/embodiment or aspects/embodiments unless clearly indicated to the contrary. In particular, any feature indicated as being preferred or advantageous may be combined with any other feature or features indicated as being preferred or advantageous.

The present invention provides a composition for topical application as defined in claim 1. A composition for topical application is defined herein as a composition that is suitable for direct application to a part of the human or animal body. Preferably, the composition is suitable for direct application to the skin, for example the face, scalp, feet, limbs or trunk.

The composition comprises a continuous aqueous phase and a discontinuous liquid oil phase. In other words, the composition comprises a dispersion of a discontinuous liquid oil phase in a continuous aqueous phase. The phases are physically distinct. The continuous aqueous phase comprises water, although it may, in addition, comprise a polar water-miscible solvent such as those described herein. The inclusion of a continuous aqueous phase in the present composition enables it to be provided in the form of a lotion or cream, as opposed to an ointment. Thus, the present composition has an improved aesthetic profile relative to the ointments of the prior art, thereby improving patient compliance. Preferably, the composition is in the form of a lotion or cream.

It is to be understood that the discontinuous liquid oil phase is homogeneous. In other words, it contains no solid inclusions or precipitates. The discontinuous liquid oil phase is a liquid at a temperature of 25 °C. Preferably, the discontinuous liquid oil phase is a liquid at a temperature of 25 °C and a pressure of 10⁵ Pa. Preferably, the discontinuous liquid oil phase has a melting point of less than 22 °C, more preferably less than 20 °C, at 1 atm pressure.

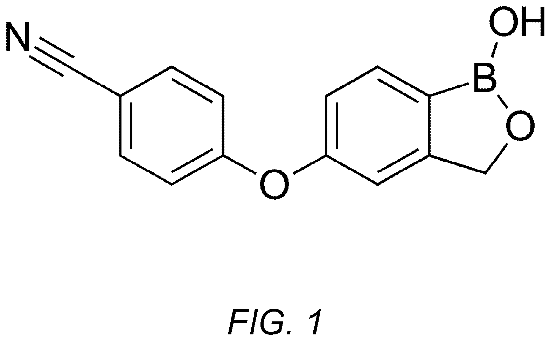

The composition comprises crisaborole. The chemical structure of crisaborole is depicted in Figure 1. The source of crisaborole is preferably anhydrous crisaborole, although it will be appreciated that other sources of crisaborole may be used. In particular, the term "crisaborole" as used herein encompasses the free acid, salts (such as pharmaceutically acceptable salts), solvates and hydrates thereof. However, the amounts of crisaborole to be incorporated into the compositions described herein are based on the anhydrous form of crisaborole. It would be within the capabilities of the skilled person to adjust the quantity used in the preparation of the composition depending on the source used to provide the desired amount in the final composition.

Preferably, the crisaborole is present in an amount of from 1 to 5 wt% by weight of the composition, more preferably from 1.5 to 5 wt%, still more preferably from 1.5 to 4 wt%, and most preferably from 1.5 to 3.5 wt%. The present inventors have found that the present invention enables crisaborole to remain fully solvated in the composition even when it is present at relatively high concentrations. Preferably at least 95 wt% of the crisaborole is dissolved in the composition, more preferably at least 98 wt%, and most preferably at least 99 wt%. In other words, preferably at least 95 wt% of the crisaborole is in solution in the composition, more preferably at least 98 wt%, and most preferably at least 99 wt%. Preferably, the crisaborole is dissolved at these levels throughout a temperature range of from 4 to 20°C.

Preferably, the crisaborole is predominantly in the discontinuous liquid oil phase. By "predominantly" it is meant that at least 90 wt% of the crisaborole in the composition is present in the discontinuous liquid oil phase, preferably at least 95 wt%, and more preferably at least 99 wt%.

One strategy developed by the inventors for achieving good solvation of the crisaborole at high crisaborole concentrations is to use a high oil loading and/or a particular combination of oils to solvate the crisaborole in the discontinuous liquid oil phase. Thus, in some embodiments, the discontinuous liquid oil phase is present in an amount of at least 40 wt% by weight of the composition, preferably at least 50 wt%, more preferably at least 60 wt%, still more preferably at least 65 wt%, and most preferably at least 70 wt%. Preferably, the discontinuous liquid oil phase is present in an amount of at most 80 wt% by weight of the composition. In these embodiments, the crisaborole is preferably predominantly in the discontinuous liquid oil phase.

The discontinuous liquid oil phase comprises an oil, preferably a pharmaceutically acceptable oil. Preferably, the oil is present in an amount of at least 40 wt% by weight of the composition, preferably at least 50 wt%, preferably at least 60 wt%, still more preferably at least 65 wt%, and most preferably at least 70 wt%. Preferably, the oil is present in an amount of at most 80 wt% by weight of the composition. In these embodiments, the continuous aqueous phase preferably comprises less than 10 wt%, preferably less than 5 wt%, more preferably less than 2 wt%, and most preferably less than 1 wt% by weight of the composition of a polar water-miscible solvent selected from the group consisting of C₁-C₄ alcohols, polyethylene glycol, ethylene glycol, propylene glycol, butylene glycol, pentylene glycol, glycerol, diethylene glycol mono ethyl ether, propylene carbonate and mixtures of two or more thereof.

Examples of oils which may be used in the present invention include coconut oil, squalane, isopropyl myristate, isopropyl isostearate, isopropyl palmitate, modified triglycerides, caprylic capric glycerides, fractionated triglycerides, glyceryl tricaprate, glyceryl tricaproate, glyceryl tricaprylate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate, linoleic glycerides, saturated polyglycolized glycerides, synthetic medium chain triglyceride containing primarily C₈-C₁₂ fatty acid chains, medium chain triglycerides, long chain triglycerides, modified triglycerides, fractionated triglycerides, isostearyl isostearate, mineral oil, dimethicone, cyclomethicone, hydrogenated polyisobutene, heptamethylnonane, and mixtures thereof. Further examples of oils which may be used in the present invention include castor oil, oleic acid, oleyl alcohol, and mixtures thereof, optionally in a mixture with one or more of the aforementioned oils.

In some embodiments described herein but not claimed, the oil comprises diisopropyl adipate and/or diethyl sebacate and/or dibutyl sebacate. In these embodiments, the diisopropyl adipate and/or diethyl sebacate and/or dibutyl sebacate are preferably present in a total amount of at least 60 wt% by weight of the discontinuous liquid oil phase, more preferably at least 70 wt%, still more preferably at least 75 wt%. Preferably, the diisopropyl adipate and/or diethyl sebacate and/or dibutyl sebacate are present in a total amount of at most 80 wt% by weight of the discontinuous liquid oil phase. Preferably the oil comprises diisopropyl adipate, more preferably in the aforementioned amounts. These preferred oils have been found to have a good solubility profile for crisaborole. Nevertheless, it is known to be difficult to obtain stable dispersions where these oils are used as a high proportion of the oil phase. Surprisingly, the present inventors have found that these oils can be incorporated as a high proportion of the discontinuous liquid oil phase.

In these embodiments, in addition to the diisopropyl adipate and/or diethyl sebacate and/or dibutyl sebacate, the oil preferably further comprises caprylic/capric triglycerides and/or castor oil, more preferably castor oil. The caprylic/capric triglycerides and/or castor oil increase the viscosity of the diisopropyl adipate and/or diethyl sebacate and/or dibutyl sebacate, thereby improving its processability. It may also improve the physical stability of the composition especially when low levels of surfactant are used. Surprisingly and unexpectedly, the inclusion of caprylic/capric triglycerides and/or castor oil has been found to provide improved dermal diffusion of the active. In some embodiments, the oil comprises or consists of diisopropyl adipate and castor oil.

According to the present invention, the oil comprises diisopropyl adipate and/or diethyl sebacate and/or dibutyl adipate. The diisopropyl adipate and/or diethyl sebacate and/or dibutyl adipate are preferably present in a total amount of at least 60 wt% by weight of the discontinuous liquid oil phase, more preferably at least 70 wt%, still more preferably at least 75 wt%. Preferably, the diisopropyl adipate and/or diethyl sebacate and/or dibutyl adipate are present in a total amount of at most 80 wt% by weight of the discontinuous liquid oil phase. Preferably the oil comprises diisopropyl adipate, more preferably in the aforementioned amounts. These preferred oils have been found to have a good solubility profile for crisaborole and achieve good dermal diffusion of the active in an *in vitro* model, as shown in Example 6. Nevertheless, it is known to be difficult to obtain stable dispersions where these oils are used as a high proportion of the oil phase. Surprisingly, the present inventors have found that these oils can be incorporated as a high proportion of the discontinuous liquid oil phase in the present invention.

In addition to the diisopropyl adipate and/or diethyl sebacate and/or dibutyl adipate, the oil preferably further comprises caprylic/capric triglycerides and/or castor oil, more preferably castor oil. The effects of these additional oils are explained above. Preferably, the oil consists essentially of or consists of the diisopropyl adipate and/or diethyl sebacate and/or dibutyl adipate, and, where present, the caprylic/capric triglycerides and/or castor oil are preferably present in a total amount of at least 10 wt% by weight of the discontinuous liquid oil phase, preferably at least 25 wt%.

In certain especially preferred embodiments, the oil comprises, by weight of the composition:
(i) diisopropyl adipate in an amount of from 5 to 45 wt%;
(ii) diethyl sebacate and/or dibutyl adipate in a total amount of from 5 to 45 wt%; and
(iii) castor oil and/or caprylic/capric triglycerides in a total amount of from 5 to 45 wt%.

Preferably the oil consists essentially of or consists of components (i) to (iii), preferably in the aforementioned amounts.

In certain especially preferred embodiments, the oil comprises, by weight of the composition:
(i) diisopropyl adipate in an amount of from 5 to 45 wt%;
(ii) diethyl sebacate in a total amount of from 5 to 45 wt%; and
(iii) castor oil in a total amount of from 5 to 45 wt%.

Preferably the oil consists essentially of or consists of components (i) to (iii), preferably in the aforementioned amounts.

In certain especially preferred embodiments, the oil comprises, by weight of the composition:
(i) diisopropyl adipate in an amount of from 5 to 33 wt%;
(ii) diethyl sebacate and/or dibutyl adipate in a total amount of from 5 to 33 wt%; and
(iii) castor oil and/or caprylic/capric triglycerides in a total amount of from 5 to 33 wt%.

Preferably the oil consists essentially of or consists of components (i) to (iii), preferably in the aforementioned amounts.

In certain especially preferred embodiments, the oil comprises, by weight of the composition:
(i) diisopropyl adipate in an amount of from 5 to 33 wt%;
(ii) diethyl sebacate in a total amount of from 5 to 33 wt%; and
(iii) castor oil in a total amount of from 5 to 33 wt%.

Preferably the oil consists essentially of or consists of components (i) to (iii), preferably in the aforementioned amounts.

Preferably, the composition of the present invention comprises at least 10 wt% water by weight of the composition, more preferably at least 20 wt%. Preferably, the composition comprises at most 60 wt% water by weight of the composition, more preferably at most 40 wt%.

Preferably, the composition of the present invention comprises a surfactant. The surfactant may be incorporated into the discontinuous liquid oil phase and/or the continuous aqueous phase. Suitable surfactants include an alkyl polyglycol ether, an alkyl polyglycol ester, an ethoxylated alcohol, a polyoxyethylene sorbitan fatty acid ester, a polyoxyethylene fatty acid ester, an ionic or non-ionic surfactant, a hydrogenated castor oil/polyoxyethylene glycol adduct containing from 25 to 60 ethoxy groups, a castor oil/polyoxyethylene glycol adduct containing from 25 to 45 ethoxy groups, a sorbitan fatty acid ester (for example Span 20 or Span 80), a block copolymer of ethylene oxide and propylene oxide (for example Pluronic L121 or Pluronic F68), or a mixture thereof. Alternatively a surfactant comprising a crosslinked copolymer can be used, the crosslinked copolymer comprising acrylic acid monomer units. Examples of these include Pemulen Tr-1 and Pemulen Tr-2 (Lubrizol Corporation). It will be understood that other suitable surfactants may be used.

Preferably, the composition comprises two or more surfactants, for example a first surfactant incorporated into the discontinuous liquid oil phase, and a second, different surfactant incorporated into the continuous aqueous phase. The first and second surfactants are preferably selected from the list above. The first surfactant readily dissolves or disperses in the discontinuous liquid oil phase and is preferably selected from the group consisting of Laureth-4 (polyoxyethylene (4) monododecyl ether), polysorbate 80, Span 80, Pemulen Tr-2 and mixtures of two or more thereof. The second surfactant readily dissolves or disperses in the continuous aqueous phase and is preferably selected from the group consisting of Polysorbate 20, Pluronic L121, Pluronic F68, PEG-40 hydrogenated castor oil, Span20, Pemulen Tr-2 and mixtures of two or more thereof. Most preferably, the first surfactant is Laureth-4 (polyoxyethylene (4) monododecyl ether), and the second surfactant is Polysorbate 20.

Preferably, the composition has a total surfactant content of less than 5 wt% by weight of the composition, more preferably less than 3 wt%. Preferably, the total surfactant content is at least 0.1 wt%. The use of lower levels of surfactant minimises the skin irritancy caused by the composition. This is especially advantageous where the patient already has inflamed skin.

The composition comprises less than 5 wt% by weight of the composition, more preferably less than 2 wt%, and most preferably less than 1 wt%, of glyceryl monostearate, cetyl alcohol, stearyl alcohol, stearic acid, cetyl ricinoleate, propylene glycol stearate, PEG-2 stearate, sorbitan monostearate, or mixtures of two or more thereof. It is to be understood that these components do not need to be present in the composition but that, if they are, their combined amount must be less than 5 wt%. Advantageously, it has been found that avoiding the use of these solidifying components serves to improve the skin penetration of the crisaborole.

Preferably, the composition comprises less than 5 wt% by weight of the composition, more preferably less than 2 wt%, and most preferably less than 1 wt%, of a wax component that is solid at 25 °C. Advantageously, it has been found that avoiding the use of such a solidifying component serves to improve the skin penetration of the crisaborole.

Preferably, the composition further comprises a second discontinuous liquid oil phase. In this embodiment, the second discontinuous liquid oil phase is physically distinct from the first discontinuous liquid oil phase. The second discontinuous liquid oil phase may, for example, comprise agents such as emollient oils (to improve in use 'feel'), occlusive oils to prevent skin dehydration and to enhance skin permeation by the active, agents that provide a heating or cooling sensation when applied to the skin or sunscreens.

Preferably, the second discontinuous liquid oil phase comprises or consists of mineral oil. Preferably, the second discontinuous liquid oil phase is present in an amount of from 10 to 30 wt% by weight of the composition, more preferably from 15 to 25 wt%.

Preferably, the composition comprises one or more further pharmaceutically active agents. It is to be understood that the term "further pharmaceutically active agent" encompasses the agent itself as well as salts (such as pharmaceutically acceptable salts), prodrugs, esters, solvates and hydrates thereof. The further pharmaceutically active agent may be useful for the treatment of atopic dermatitis or psoriasis. Examples of further pharmaceutically active agents for use in combination with crisaborole include but are not limited to:
Topical corticosteroids such as Fluocinonide, Desoximetasone, Mometasone, Triamcinolone, Betamethasone, Betamethasone Diproprionate, Alclometasone, Desonide, Hydrocortisone and Mapracorat;
Topical Calcineurin inhibitors such as Tacrolimus, pimecrolimus and cyclosporine;
Topical PDE4 inhibitors such as apremilast and Roflumilast;
Topical JAK kinase inhibitors such as Tofacitinib, Baricitinib and Upadacitinib; and
Vitamin D analogs such as calcipotriene.

Providing the crisaborole and the one or more further pharmaceutically active agents in a single composition allows for the simultaneous administration of crisaborole and the further pharmaceutically active agent(s).

Preferably, the composition further comprises one or more pharmaceutically acceptable excipients. Suitable excipients for topical compositions are known in the art and include agents that repair any skin damage that results from the application of the composition.

As noted above, the composition is in the form of an oil-in-aqueous dispersion. Preferably, the composition is in the form of an emulsion. Emulsions are known in the art.

In an alternative aspect not according to the present invention, the composition may be in the form of a polyaphron dispersion. By polyaphron dispersion as used herein it is meant a particular kind of hydrophilic liquid-in-hydrophobic liquid or hydrophobic liquid-in-hydrophilic liquid dispersion comprising (a) a hydrophilic liquid miscible phase, (b) a second hydrophobic phase being immiscible or substantially immiscible with the first phase and (c) one or more surfactants, wherein the dispersed or discontinuous liquid oil phase is in the form of small (e.g. micron to sub-micron diameter, but more usually at least 1 micron diameter) droplets, and the whole having the following characteristics, which distinguish polyaphron dispersions from conventional or common emulsions and other dispersion types:
1. They are capable of existing in a stable form wherein the volume fraction of the dispersed phase (ϕᵢₚ) is greater than 0.7 and can be as high as 0.97. (ϕᵢₚ is the volume ratio of discontinuous to continuous aqueous phase expressed as a fraction).
2. The microscopic appearance of polyaphron dispersions where ϕᵢₚ is greater than 0.7 is that of an aggregate of individual droplets, pushed closely together into polyhedral shapes, resembling the appearance of a gas foam. In this form, the dispersion has gel-like properties and is referred to as a Gel Polyaphron Dispersion (GPD).
3. Stable polyaphron dispersions can be formed with a surfactant concentration less than 3% and more typically less than 2% by weight of the total composition.
4. Gel Polyaphron Dispersions (as described in 2 above) can be diluted to any extent by the addition of more continuous aqueous phase without the addition of more surfactant, when the gel-like properties disappear. Once ϕᵢₚ has been reduced to below 0.7, the individual droplets of internal phase become separated to take the form of spherical droplets, which remain stable and intact but which may nevertheless join together in loose associations and float to the top or sink to the bottom of the diluted dispersion (depending on the relative densities of the two phases). In this diluted form each droplet is referred to as a Colloidal Liquid Aphron (CLA). Simple shaking of the diluted dispersion instantly causes a homogeneous, stable dispersion of Colloidal Liquid Aphrons to reform.

Polyaphron dispersions are disclosed in the following literature references by Sebba: "Biliquid Foams", J. Colloid and Interface Science, 40 (1972) 468-474 and "The Behaviour of Minute Oil Droplets Encapsulated in a Water Film", Colloid Polymer Sciences, 257 (1979) 392-396, Hicks "Investigating the Generation, Characterisation, and Structure of Biliquid Foams", PhD Thesis, University of Bristol, 2005, Crutchley "The Encapsulation of Oils and Oil Soluble Substances Within Polymer Films", PhD Thesis, The University of Leeds, 2006 and Lye and Stuckey, Colloid and Surfaces, 131 (1998) 119-136. Aphrons are also disclosed in US-A-4,486,333 and WO 97/32559. Polyaphron dispersions are sometimes referred to as 'Biliquid Foams', 'High Internal Phase Emulsions (HIPEs)', 'High Internal Phase Ratio Emulsions (HIPREs)' and 'Gel Emulsions'. In US 5,573,757 a composition comprising a polyaphron dispersion is described as "a viscoelastic gel".

According to the present invention, the composition does not comprise a polyaphron dispersion. The composition does not comprise a polyaphron dispersion comprising a continuous aqueous phase, a discontinuous liquid oil phase and crisaborole.

Preferably, the composition of the present invention is dispersible in water. Preferably the composition of the present invention is dilutable in water. This increases the flexibility of use of the invention, for example in improving the application of the composition to the scalp through hair by leaving the hair wet, or from rinsing the preparation from any topical surface should the desire or need arise, or by the easy removal by rinsing of product from accidental contamination of clothing. These advantages improve the in-use experience of users and improve patient compliance.

Preferably, the composition of the present invention further comprises a gelling agent and/or a rheology modifying agent, such as a viscosity modifier. The gelling agent may, for example, be selected from alginate gums or their salts, guar gum, locust bean gum, xanthan gum, gum acacia, gelatin, hydroxymethylcellulose, hydroxyethylcellulose, hydroxypropylcellulose, carboxymethylcellulose or its salts, bentonites, magnesium aluminium silicates, "Carbomers" (salts of cross-linked polymers of acrylic acid), or glyceryl polymethacrylates or their dispersions in glycols. It will be understood that other suitable gelling agents may be used. Additionally, it has been found that some of the gelling agents (for example, carbomers) may also function as a chemical buffering agents thus preventing unwanted variation in the pH of the composition during storage and use. Where a viscosity modifier is used, this is preferably a polymeric cellulosic thickener. The inclusion of a gelling agent and/or rheology modifying agent provides additional stability against creaming and ensures that the active concentration is uniform throughout the composition. The use of these components is described in WO97/32559. The choice of gelling/thickening agents also allows for control of formulation viscosity from a thin lotion that is readily pourable to a thick cream with a significant resistance to flow.

Preferably, the composition of the present invention comprises from 0.05 to 5.0 wt% by weight of the composition of a gelling agent, more preferably from 0.1 to 2.0 wt% and most preferably from 0.2 to 1.0 wt%. In one embodiment of the present invention the composition has the consistency of a gel.

The compositions of the present invention may also contain other additives such as preservatives (for instance to prevent microbiological spoilage), buffering agents (for the control of pH and to avoid instability and damage to the skin's acid mantle) and antioxidants. Where a preservative is used, it is preferably present in an amount of from 0.1 to 1 wt%, more preferably 0.5 to 1 wt%, still more preferably from 0.6 to 0.8 wt%, by weight of the composition. The preservative is preferably benzyl alcohol or phenoxyethanol, more preferably phenoxyethanol. These additives may be included in the continuous or the discontinuous liquid oil phase of the composition. It will be understood that the inclusion of these additives will be at the levels and with the type of materials which are found to be effective and useful. Care needs to be taken in the choice and amount of these additives to prevent compromise to the other performance advantages of the present invention.

Preferably, the composition has a pH of from about 4 to about 6.5, more preferably from about 5 to about 6, and most preferably about 5.5. As shown in Example 7, crisaborole has optimal stability in aqueous solution within these ranges. It will be understood that any suitable acid or base may be used to adjust the pH to the appropriate value or pH range. Typically the pH of the composition will need to be raised by the addition of a base, which suitably may be triethanolamine. Other suitable bases include, but are not limited to trishydroxymethylaminomethane (tris), sodium hydroxide and potassium hydroxide. Advantageously and preferably, the pH of the composition may be stabilized by the incorporation of a suitable buffer into the aqueous phase. Suitable buffer systems having a pH within the specified range will be familiar to those skilled in the art.

Preferably, the composition is chemically stable for at least 12 months at 5 °C ± 3 °C, as measured at 60% RH ± 5%. The stability is measured after storage in a closed, airtight glass container with headspace comprising no more than 5% by volume of the total usable volume of the container. By "chemically stable" it is meant an HPLC assay for crisaborole of 100%±5% relative to the measurement at t=0.

Preferably, the composition is chemically stable for at least 12 months at 25 °C ± 2 °C, as measured at 60% RH ± 5%. The stability is measured after storage in a closed, airtight glass container with headspace comprising no more than 5% by volume of the total usable volume of the container. Again, by "chemically stable" it is meant an HPLC assay for crisaborole of 100%±5% relative to the measurement at t=0.

Preferably, the composition is chemically stable for at least 6 months at 40 °C ± 3 °C. The stability is measured after storage in a closed, airtight glass container with headspace comprising no more than 5% by volume of the total usable volume of the container. "Chemically stable" takes the same meaning as above.

Preferably, the composition is physically stable for at least 12 months at 5 °C ± 3 °C, as measured at 60% RH ± 5%. The stability is measured after storage in a closed, airtight glass container with headspace comprising no more than 5% by volume of the total usable volume of the container. By "physically stable" it is meant that the composition appears as a homogeneous cream with no gross apparent rheological or appearance changes from t=0. Preferably, by "physically stable" it is also meant that the composition does not appear to contain crystals or solid particulates when viewed under a microscope, and/or that the composition has a particle size distribution profile that has undergone no significant changes from t=0. The particle size distribution can be determined by microscopy or laser diffraction, such as using a Malvern Mastersizer 2000 or Malvern Mastersizer 3000.

Preferably, the composition is physically stable for at least 12 months at 25 °C ± 2 °C, as measured at 60% RH ± 5%. The stability is measured after storage in a closed, airtight glass container with headspace comprising no more than 5% by volume of the total usable volume of the container. Again, by "physically stable" it is meant that the composition appears as a homogeneous cream with no gross apparent rheological or appearance changes from t=0, and preferably that the composition does not appear to contain crystals or solid particulates when viewed under a microscope, and/or that the composition has a particle size distribution profile that has undergone no significant changes from t=0.

Preferably, the composition is physically stable for at least 6 months at 40 °C ± 3 °C, as measured at 60% RH ± 5%. The stability is measured after storage in a closed, airtight glass container with headspace comprising no more than 5% by volume of the total usable volume of the container. "Physically stable" takes the same meaning as above. For the stability measurements, the glass container is preferably sparged with nitrogen after the glass container has been filled with the composition but prior to the storage of the composition.

In an especially preferred embodiment a composition as defined in claim 1 specifically comprises, by weight of the composition:
from 2 to 4 wt% crisaborole, and
at least 60 wt% discontinuous liquid oil phase;
wherein the crisaborole is predominantly in the discontinuous liquid oil phase,
wherein the oil comprises at least 60 wt% diisopropyl adipate by weight of the discontinuous liquid oil phase, and
wherein the composition is in the form of a lotion or cream.

In a further especially preferred embodiment a composition as defined in claim 1 specifically comprises, by weight of
the composition:
from 2 to 4 wt% crisaborole,
at least 50 wt% discontinuous liquid oil phase, and
less than 2 wt%, and most preferably less than 1 wt%, of glyceryl monostearate, cetyl alcohol, stearyl alcohol, stearic acid, cetyl ricinoleate, propylene glycol stearate, PEG-2 stearate, sorbitan monostearate, or mixtures of two or more thereof;
wherein the crisaborole is predominantly in the discontinuous liquid oil phase,
wherein the oil comprises at least 60 wt% diisopropyl adipate by weight of the discontinuous liquid oil phase, and
wherein the composition is in the form of a lotion or cream.

In a further especially preferred embodiment a composition as defined in claim 1 specifically comprises from 1.5 to 5 wt% crisaborole by weight of the composition, wherein the crisaborole is predominantly in the discontinuous liquid oil phase, wherein the oil comprises, by weight of the composition:
(i) diisopropyl adipate in an amount of from 5 to 45 wt%;
(ii) diethyl sebacate and/or dibutyl adipate in a total amount of from 5 to 45 wt%; and
(iii) castor oil and/or caprylic/capric triglycerides in a total amount of from 5 to 45 wt%,
wherein the composition does not comprise a polyaphron dispersion.

According to a further aspect, there is provided a combination comprising a first composition in accordance with the first aspect and a second composition comprising a further pharmaceutically active agent. The further pharmaceutically active agent may be useful for the treatment of atopic dermatitis or psoriasis. Suitable examples of such agents are known to the skilled person and are described herein in relation to the first aspect. As will be appreciated, providing the crisaborole and the further pharmaceutically active agent in separate compositions allows the crisaborole and further pharmaceutically active agent to be administered sequentially. It also allows the crisaborole and further pharmaceutically active agent to be administered simultaneously, for example where the user mixes the separate compositions prior to administration.

According to a further aspect, there is provided a composition or combination as described herein for use in the treatment of the human or animal body by therapy.

According to a further aspect, there is provided a composition or combination as described herein for use in the treatment of atopic dermatitis or psoriasis.

The composition or combination as described herein can also be used to treat other inflammatory dermatological conditions.

The composition or combination as described herein may be applied to the scalp or other skin surface through hair. Preferably in this embodiment the hair is wetted (for example by use of water with or without shampoo, and then towel dried). The product may then be applied to the scalp in a suitable amount and then massaged into the scalp through the hair. The hair may then be left to dry naturally or dried using a hair dryer. Advantageously, the water-dispersible form of the formulation enables an even distribution of the actives on the skin using this process. Alternatively, or additionally, the composition or combination may be massaged into the scalp through dry hair and left for a suitable period (which may be 8 to 12 hours) after which the excess or reminder may be rinsed out with water with or without shampoo. Preferably the composition or combination is applied to a human or animal in unit dosage form.

The composition or combination is preferably administered at least once a week, more preferably at least once a day.

Preferably, the composition or combination is administered directly to the skin.

Preferably, the composition or combination is administered directly to and rubbed into the skin.

The composition or combination may be administered in the form of a spray.

Preferably, the composition or combination is administered to a human subject to provide a dosage of crisaborole of from 1 to 50 mg/kg/day, more preferably from 2 to 20 mg/kg/day. These dosages are especially suited for treating atopic dermatitis, psoriasis and/or other inflammatory dermatological conditions.

According to a further aspect, there is provided a package comprising from 20 to 300 g of the composition described herein. Preferably, the package comprises from 20 to 200 g of the composition, more preferably from 50 to 100 g. Preferably, the package is a jar, a tube, an airless pump, a sachet, a bottle, a tub, a pump action sealed container, or a spray applicator. For example, a tube can be squeezed for topical application of the composition.

In an embodiment described herein but not claimed, a method for manufacturing a composition comprises:
(i) providing a hydrophilic solvent, optionally comprising crisaborole and/or a surfactant;
(ii) providing a hydrophobic solvent, optionally comprising crisaborole and/or a surfactant; and
(iii) mixing the hydrophilic solvent with the hydrophobic solvent under suitable conditions to form a composition for topical application comprising a continuous aqueous phase, a discontinuous liquid oil phase and crisaborole.

Preferably, the composition produced by the method is the composition as described herein in relation to the first aspect. Methods for preparing such oil-in-water dispersions are known in the art, for example in G. Godwin, Harry's Cosmeticology 7^{th} Edition, 1982. It will be understood by those skilled in the art that other manufacturing methods may be used, as appropriate.

As noted in relation to the first aspect, the composition does not comprise a polyaphron dispersion. Suitable methods for preparing polyaphron dispersions are described in US-A-4486333. It will be understood by those skilled in the art that other manufacturing methods may be used, as appropriate.

Preferably, the method further comprises packaging the composition.

The foregoing aspects may be freely combined with any of the foregoing aspects disclosed herein.

The present invention will now be described in relation to the following non-limiting figures:
**Figure 1** depicts the chemical structure of crisaborole.

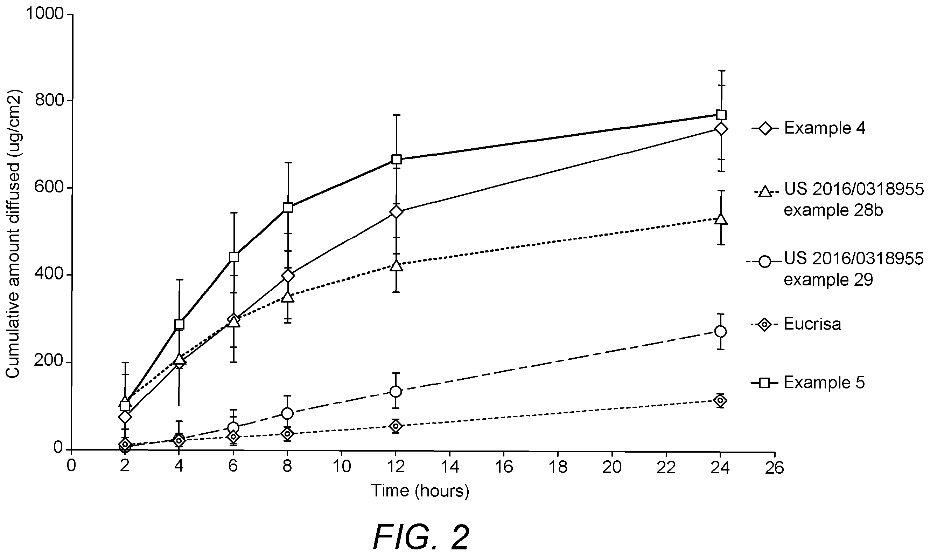

**Figure 2** is a graph showing the mean diffusion of the samples described in Reference Example 6. The y-axis indicates mean cumulative crisaborole diffused in µg/cm². The error bars indicate standard error. In the data points, the diamond (unfilled) represents Example 4 described herein but not according to the invention, the triangle represents the sample based on Example 28b of US 2016/0318955, the circle represents the sample based on Example 29 of US 2016/0318955, the diamond (filled) represents the commercially available Eucrisa^{®} ointment, and the square represents Example 5 described herein but not according to the invention.

The present invention will now be described in relation to the following non-limiting examples.

### Reference Example 1

10 grams of a composition for topical application not in accordance with the present invention was prepared by combining the following components:
8.75 g polyaphron dispersion
1.1 g gel mix
0.15 g NaOH (20% aq)
water q.s.

The polyaphron dispersion had the following composition:

| | **Wt in final (g)** | **% in final** |
|---|---|---|
| **Oil Phase** | | |
| Crisaborole | 0.20 | 2.00 |
| Diisopropyl adipate | 5.00 | 50.00 |
| Capric/caprilic triglyceride | 2.00 | 20.00 |
| Laureth-4 | 0.07 | 0.70 |
| | | |

| **Aqueous phase** | | |
|---|---|---|
| Polysorbate 20 | 0.015 | 0.15 |
| Water | 1.46 | 14.60 |
| | 8.745 | 87.45 |

The polyaphron dispersion was prepared by mixing the oil phase components together in a vessel using 40°C heating and stirred with a magnetic follower until fully dissolved. The mixture was then allowed to cool to room temperature. In a separate vessel the aqueous phase components were mixed using an overhead stirrer with a propeller style blade at 70rpm. Once homogeneous the stirrer speed was increased to 300rpm and the oil phase was slowly added over 20minutes. The system was mixed for a further 20minutes to ensure an even droplet sized distribution. The final polyaphron dispersion had the appearance of a white viscous cream.

The gel mix had the following composition:

| | Quantity (g) | Wt in final (g) | % in final |
|---|---|---|---|
| Carbomer (Ultrez 10 | 2.08 | 0.02288 | 0.2288 |
| Natrosol 250L | 0.42 | 0.00462 | 0.0462 |
| Phenoxyethanol | 1.46 | 0.01606 | 0.1606 |
| Water | 96.04 | 1.05644 | 10.5644 |
| | 100.00 | 1.100 | 11.00 |

The Natrosol, Phenoxyethanol and water were mixed using a magnetic follower and 40°C heating until the Natrosol had fully dissolved. Still stirring the system was then allowed to cool and the carbomer was added. Mixing was continued until the carbomer was fully dispersed with no evidence of lumps.

The gel mix was then added to the polyaphron dispersion and mixed using an overhead stirrer (70rpm) for 5 minutes before neutralization with the sodium hydroxide solution.

The resulting composition appeared to be stable with no signs of crystals under polarized light.

### Reference Example 2

A composition for topical application comprising the following components and not in accordance with the present invention was prepared:

| | | **Quantity (g)** | **% in final** |
|---|---|---|---|
| Polyaphron dispersion | **Oil phase** | | |
| | Mineral oil | 50.00 | 50.00 |
| | | | |
| | **Aqueous phase** | | |
| | Cremophor RH40 | 0.50 | 0.50 |
| | Propylene glycol | 7.80 | 7.80 |
| | Water | 4.20 | 4.20 |
| | | 62.50 | 62.50 |
| | | | |
| | | | |
| Gel phase | Crisaborole | 2.00 | 2.00 |
| | Propylene glycol | 24.70 | 24.70 |
| | Carbomer (Ultrez 10) | 0.50 | 0.50 |
| | Water | 12.00 | 12.00 |
| | | | |
| Adjust | Trolamine (50%a.q.) | q.s. | q.s. |
| | Water | q.s. | q.s. |
| | | 100.00 | 100.00 |

The polyaphron dispersion was made using the method of Reference Example 1.

The gel phase was made by dispersing the carbomer in the water using a magnetic follower until the dispersion was fully solvated and free of lumps. In a separate vessel the crisaborole was dissolved in the propylene glycol using a magnetic follower. Under suitable mixing the two are then combined.

The polyaphron dispersion was then mixed into the gel phase and the trolamine/water was added to increase the pH to activate the thickener, thereby thickening the system.

The resulting composition appeared to be stable with no signs of crystals under polarized light.

### Reference Example 3

A composition for topical application comprising the following components and not in accordance with the present invention was prepared:

| | | **Quantity (g)** | **% in final** |
|---|---|---|---|
| API solution | Crisaborole | 2.00 | 2.00 |
| | Diethylene glycol monoethyl ether | 20.00 | 20.00 |
| | | | |
| Gel phase | Carbomer (Ultrez 10) | 0.50 | 0.50 |
| | Water | 15.00 | 15.00 |
| | Propylene glycol | 15.00 | 15.00 |
| | | | |
| Polyaphron dispersion | **Oil phase** | | |
| | Mineral oil | 19.34 | 19.34 |
| | Arlamol HD | 19.34 | 19.34 |
| | | | |
| | **Aqueous phase** | | |
| | Kolliphor RH40 | 0.387 | 0.387 |
| | Diethylene glycol monoethyl ether | 3.23 | 3.23 |
| | Water | 3.20 | 3.20 |
| | | | |
| Adjust (to pH5.5) | Neutrol TE (50%a.q.) | q.s. | q.s. |
| | Water | q.s. | q.s. |
| | | 100.00 | 100.00 |

The API solution was made by simple stirring with a magnetic follower (no heat required).

The gel phase was made by adding the carbomer to the water with gentle stirring until the carbomer was fully dispersed before addition of the propylene glycol.

The polyaphron dispersion was made as described in Reference Example 1.

The final system was made by slowly adding the gel phase to the API solution, followed by the polyaphron dispersion, followed by the adjust phase. All carried out under moderate stirring.

The sample was stored in a closed, airtight glass container with headspace comprising no more than 5% by volume of the total usable volume of the container. Each container containing a sample was stored at a constant temperature of 40 °C in a standard laboratory incubator (for example, Memmert IF260PLUS Incubator). The storage period for each sample was three months.

The chemical stability of the crisaborole in each sample after the storage period was measured by a HPLC method. The HPLC method was as follows:

| | | | |
|---|---|---|---|
| UPLC System | Waters Photodiode Array Detector | | |
| | Waters Acquity H-Class UPLC System | | |
| | Waters Empower3 Data Processing Software | | |
| Column | Waters Acquity UPLC CSH C18 1.7um 2.1x100mm | | |
| Guard Column | N/A | | |
| Detection | 251 nm | | |
| Sample Temperature | 5 °C | | |
| Column Temperature | 40 °C | | |
| Flow Rate | 0.5 mL/min | | |
| Mobile Phase | Mobile Phase A: 0.2% Trifluoroacetic acid in Deionised Water | | |
| | Mobile Phase B: 100% Acetonitrile | | |
| Gradient | Time (min) | % A | % B |
| | 0 | 85 | 15 |
| | 1 | 85 | 15 |
| | 5 | 70 | 30 |
| | 25 | 60 | 40 |
| | 27 | 60 | 40 |
| | 27.1 | 85 | 15 |
| | 32 | 85 | 15 |
| Injection Volume | 2 µL | | |
| Run Time | 32 min | | |

### Mobile phase preparation:

Mobile phase A: 2 mL of trifluoroacetic acid in 1 L of deionised water. Mix well before use.

### Mobile phase B: 100% Acetonitrile

Sample preparation for 2% strength formulation:
   Acetonitrile is used as the sample diluent
   Prepare all samples in amber glassware
   Procedure:
      1. Weigh 0.1 g (±0.012 g) of sample into 20 mL amber volumetric flask, minimizing sample on the neck of the flask.
      2. Add diluent to volume, and mix by inversion.
      3. Add a magnetic stirrer and stir for 1 hour at 400 rpm.
      4. Filter through 0.45 µm PTFE syringe filter into amber HPLC vials.
Standard preparation:
   Acetonitrile is used as the sample diluent
   Prepare all standards in amber glassware
   A solution of crisaborole in acetonitrile at a target concentration of 100 µg/mL

For Reference Example 3, the crisaborole peak purity (*i.e.* the area of the peak of interest compared to the total area of all API related peaks in the chromatogram, expressed as a percentage) measured after storage for 3 months at 40 °C (see above) was 97.1%.

### Reference Example 4

A composition for topical application comprising the following components and not in accordance with the present invention was prepared:

| | | **Quantity (g)** | **% in final** |
|---|---|---|---|
| | | | |
| Polyaphron dispersion | **Oil phase** | | |
| | Crisaborole | 0.80 | 2.00 |
| | Diisopropyl adipate | 6.34 | 15.84 |
| | Diethyl sebacate | 6.33 | 15.83 |
| | Castor oil | 6.33 | 15.83 |
| | Butylated hydroxyanisole | 0.04 | 0.10 |
| | Laureth 4 | 0.20 | 0.50 |
| | | | |
| | **Aqueous phase** | | |
| | Poloxamer 407 | 0.25 | 0.62 |
| | Water | 4.71 | 11.78 |
| | | | |
| | | | |
| Unneutralised Gel | Carbomer Ultrez 10 | 0.30 | 0.75 |
| | Natrosol 250L | 0.08 | 0.20 |
| | Phenoxyethanol | 0.28 | 0.70 |
| | Water | 13.54 | 33.85 |
| | | | |
| | Sodium citrate dehydrate | 0.05 | 0.123 |
| | Citric acid (anhydrous) | 0.03 | 0.071 |
| | Water | 0.32 | 0.81 |
| | | | |
| Adjust (to pH5.5) | Sodium hydroxide (20% aqueous solution) | q.s. | q.s. |
| | Water | q.s. | q.s. |
| | | 40.00 | 100.00 |

The composition was prepared using an analogous method to Reference Example 1.

The crisaborole peak purity measured after storage for 3 months at 40 °C under the conditions described in Reference Example 3 was 99.0%.

### Reference Example 5

A composition for topical application comprising the following components and not in accordance with the present invention was prepared:

| | | **Quantity (g)** | **% in final** |
|---|---|---|---|
| | | | |
| Polyaphron dispersion | **Oil phase** | | |
| | Crisaborole | 0.80 | 2.00 |
| | Diisopropyl adipate | 7.60 | 19.00 |
| | Diethyl sebacate | 7.60 | 19.00 |
| | Capric/caprylic triglycerides (Miglyol 810 ^{™}) | 3.80 | 9.50 |
| | Butylated hydroxyanisole | 0.04 | 0.10 |
| | Laureth 4 | 0.20 | 0.50 |
| | | | |
| | **Aqueous phase** | | |
| | Poloxamer 407 | 0.25 | 0.62 |
| | Water | 4.71 | 11.78 |
| | | | |
| | | | |
| Unneutralised Gel | Carbomer Ultrez 10 | 0.30 | 0.75 |
| | Natrosol 250L | 0.08 | 0.20 |
| | Phenoxyethanol | 0.28 | 0.70 |
| | Water | 13.54 | 33.85 |
| | | | |
| | Sodium citrate dehydrate | 0.05 | 0.123 |
| | Citric acid (anhydrous) | 0.03 | 0.071 |
| | Water | 0.32 | 0.81 |
| | | | |
| Adjust (to pH5.5) | Sodium hydroxide (20% aqueous solution) | q.s. | q.s. |
| | Water | q.s. | q.s. |
| | | 40.00 | 100.00 |

The composition was prepared using an analogous method to Reference Example 1.

The crisaborole peak purity measured after storage for 3 months at 40 °C under the conditions described in Reference Example 3 was 99.3%.

### Reference Example 6

Certain formulations were tested in a Franz cell *in vitro* diffusion experiment though an artificial membrane. The membrane used was a Strat-M Membrane from Merck Millipore and is described as a synthetic, non-animal based model for transdermal diffusion testing that is predictive of diffusion in human skin. A solution of 75% propylene glycol and 25% water was used as the receptor phase. Cells with a surface area of 0.64 cm² and a receptor volume of approximately 2 cm³ were used. Approximately 30 mg of formulation was applied at time 0 hours and the cells were stirred and incubated at 37 °C throughout. The receptor phase was sampled and replaced at 2, 4, 6, 8, 12 and 24 hours. Crisaborole concentration was determined by HPLC using a similar method to that of Reference Example 3 but using the following parameters:

| | | | |
|---|---|---|---|
| UPLC System | Waters Photodiode Array Detector | | |
| | Waters Acquity H-Class UPLC System | | |
| | Waters Empower3 Data Processing Software | | |
| Column | Waters Acquity UPLC CSH C18 1.7um 2.1x100mm | | |
| Guard Column | N/A | | |
| Detection | 251 nm | | |
| Sample Temperature | 5 °C | | |
| Column Temperature | 40 °C | | |
| Flow Rate | 0.5 mL/min | | |
| Mobile Phase | Mobile Phase A: 0.2% Trifluoroacetic acid in Deionised Water | | |
| | Mobile Phase B: 100% Acetonitrile | | |
| Gradient | Time (min) | % A | % B |
| | 0 | 68 | 32 |
| | 5 | 68 | 32 |
| Injection Volume | 2 µL for StratM diffusion | | |
| Run Time | 5 min | | |

Mean cumulative crisaborole per cm² of membrane for each sample was then plotted (see Figure 2). The formulations tested were Examples 4 and 5 described herein but not according to the invention, two formulations prepared in accordance with US 2016/0318955 (Examples 28B and 29, but with the boron-based active agent replaced with crisaborole) and a commercially available Eucrisa^{®} ointment (all having a crisaborole content of 2.0 wt%).

It can be seen from Figure 2 that the liquid oil-phased formulations (Examples 4 and 5 described herein) are superior in terms of *in vitro* diffusion properties to the formulations based on those disclosed in US 2016/0318955 and the Eucrisa^{®} ointment.

### Reference Example 7

A 1% solution of crisaborole was prepared with 50% ethanol and a citrate buffer to pH 5.5. The system was then stored at 40°C for one month under the conditions of Reference Example 3 and gave an 84% peak purity. Equivalent solutions were prepared and tested at pH 4.5, 6,5, 7.5 and 8.5. The results are shown in the following table:

| **pH** | **% peak purity** |
|---|---|
| 4.5 | 79.5 |
| 5.5 | 84.1 |
| 6.5 | 79.8 |
| 7.5 | 72.1 |
| 8.5 | 51.3 |

The results show that crisaborole is vulnerable to degradation when exposed to an aqueous medium and that its chemical stability is highest at pH 5.5.

### Reference Example 8

The formulations of Example 5 described herein, but not according to the invention, and crisaborole equivalents of

Examples 28B and 29 of US 2016/0318955 (see Reference Example 6) were tested for their chemical and physical stability. The samples were stored at room temperature for 24 hours in a closed, airtight glass container with headspace comprising no more than 5% by volume of the total usable volume of the container. The samples were then analysed using the HPLC method of Reference Example 3. The percentages of two impurity peaks are shown in the following table:

| | **Percentage area of impurity** | |
|---|---|---|
| | **RRT 0.73** | **RRT 0.92** |
| US 2016/0318955 Example 28B | 0.07 | 1.34 |
| US 2016/0318955 Example 29 | 1.02 | 0.65 |
| Example 5 | 0.06 | 0.27 |
| Standard | - | 0.02 |

As shown in the table, the sample based on Example 29 of US 2016/0318955 was the most unstable sample tested with not only additional impurities but also having crystals evident under the microscope. The two observations may be related as the crystals will be exposed to water as they are not encapsulated within the oil phase (c.f. Reference Example 7).

This may explain why the impurity at relative retention time (RRT) 0.73 is significantly higher than the other formulas. The impurity detected at 0.73RRT is believed to be a degradation impurity related to aqueous exposure.

The sample based on Example 28B of US 2016/0318955 was found to exhibit a high level of the impurity at RRT 0.92. The cause of this impurity is, at present, unknown.

In parallel, samples were stored for 2 months at 4 °C. For the sample based on Example 28B of US 2016/0318955 (crisaborole equivalent), amorphous solid lumps were observed to develop over time when examined under a microscope. These lumps were significantly different in appearance to those observed almost immediately in the sample based on Example 29 of US 2016/0318955 (see above). These amorphous solid lumps were not observed in the other samples.

### Example 9

An emulsion composition for topical application comprising the following components was prepared:

| | **Wt %** |
|---|---|
| Crisaborole* | 2.0 |
| Methylparaben | 0.15 |
| Propylparaben | 0.03 |
| Glyceryl monostearate SE* | - |
| Laureth-4* | 0.30 |
| Butylated Hydroxytoluene* | 0.02 |
| Edetate Disodium | 0.05 |
| Pemulen TR-2 | 0.25 |
| Carbopol Ultrez 10 | 0.20 |
| 25% Trolamine | 0.84 |
| Propylene glycol | 5.0 |
| Octyldodecanol* | 10.0 |
| Oleyl alcohol* | 10.0 |
| Benzyl Alcohol* | 2.0 |
| Diisopropyl Adipate* | 10.0 |
| Purified Water | QS 100 |
| | |
| ***oil phase components** | |

The method for preparing the composition was as follows:
In a suitable vessel at 60°C±5 purified water, methylparaben, propylparaben, propylene glycol and edetate disodium were combined.

In a second suitable vessel the oleyl alcohol, benzyl alcohol, diisopropyl adipate, octyldodecanol, butylated hydroxytoluene, Laureth-4 and crisaborole were combined at 60°C±5 until a clear solution was obtained. The Pemulen TR-2 was rapidly dispersed into the oil phase and then the oil phase was quickly mixed into the aqueous phase with homogenizer mixing. The temperature was maintained at 60°C±5 for 5 minutes. The mixture was allowed to cool whilst stirring with a propeller, the trolamine solution was added and mixing was continued until the composition was at room temperature.

### Example 10

A local skin penetration study in minipigs was conducted to assess the local skin penetration of the composition prepared in accordance with Examples 4 and 9 above and a composition prepared in accordance with Example 28B of US 2016/0318955 (but with the boron-based active agent replaced with 2 wt% crisaborole). In particular, the study was conducted to assess the dermal absorption of the crisaborole after being administered twice daily by dermal application on areas of 2.5 x 2.5 cm to minipigs for days. The formulation of Example 4 is not according to the invention. The formulation of Example 9 is according to the invention.

### Method:

Four (4) female Göttingen minipigs were used in study. 16 application sites each measuring 2.5 x 2.5 cm were tattooed on the back of each animal, and each of the three formulations described above was applied in 8 different application sites on each animal.

The animals were dosed according to the following schedule:

| **Treatment** | **Dose formulation amount** | **Dose formulatio n per applicatio n site** | **Total daily dose** | **No. animals** |
|---|---|---|---|---|
| | **(mg/cm²)** | **(mg)** | **(mg/animal/d ay)** | |
| Example 4 (not in accordance with the invention) | 25.0 | 156.25 | 312.5 | 4 |
| Example 9 (in accordance with the invention) | 25.0 | 156.25 | 312.5 | 4 |
| US 2016/0318955 Example 28B | 25.0 | 156.25 | 312.5 | 4 |

All formulations contained 2 wt% crisaborole.

The following were evaluated: mortality, clinical signs, body weight and necropsy with macroscopic observations of treated and untreated skin and histopathology. Skin biopsies for bioanalysis were collected on Day 8. Furthermore, application sites were examined for reaction to treatment and scored for erythema, oedema and other dermal reactions.

Biopsies were taken for bioanalysis from all application sites 4 hours +/- 10 minutes after the wash on Day 8. At each sampling time, two (one for back-up purposes) biopsies from each application site were taken. In total 24 biopsies from each animal at each sampling time. The biopsies were taken from the centre of the application sites. Prior to collection of the skin samples, the stratum corneum was separated from the skin in the part of the application site, where the biopsies were taken, by 20 strippings per two biopsies, using a commercial adhesive tape.

The biopsies were collected using a punch with a diameter of 5 mm. From the biopsies the epidermis was separated from dermis in the best possible manner using a scalpel. Following this, subcutaneous tissue was removed from the dermis using a scalpel, and then discarded. The biopsies were weighed and immediately snap frozen using liquid nitrogen and stored at -80°C or below until analyses. Tissue samples were analysed for levels of crisaborole using a validated bioanalytical assay.

### Results:

Level of crisaborole measured in minipig skin biopsies after twice daily application of test products for 7 days:

| | **Median concentration (ng/g)** | |
|---|---|---|
| | **Dermis** | **Epidermis** |
| Example 4 (not in accordance with the invention) | 4575 | 39001 |
| Example 9 (in accordance with the invention) | 1714 | 60117 |
| US 2016/0318955 Example 28B | 556 | 37180 |

On the basis of this study it can be concluded that the compositions of the present invention exhibit improved skin penetration *in vivo* than existing crisaborole cream formulations such as those disclosed in US 2016/0318955.

### Example 11

The formulation of Example 9 described herein and the crisaborole equivalent of Example 28B of US 2016/0318955 (see Example 10 herein) were tested for their chemical stability. The samples were stored at room temperature for 3 months in a closed, airtight glass container with headspace comprising no more than 5% by volume of the total usable volume of the container. The samples were then analysed using the HPLC method of Reference Example 3. The percentages of two impurity peaks are shown in the following table:

| | **RRT 0.73 impurity** | **RRT 0.92 impurity** | **% Assay** |
|---|---|---|---|
| Example 28B of US 2016/0318955 | 1.09 | 12.11 | 87.25 |
| Example 9 (in accordance with the invention) | 0.50 | 0.14 | 99.35 |

As shown in the table, the composition in accordance with the invention exhibited significantly improved chemical stability after storage for 3 months at room temperature than the composition of the prior art.

### Example 12

An emulsion composition for topical application comprising the following components was prepared:

| | | **Quantity (g)** | **% in final** |
|---|---|---|---|
| Dispersion | **Oil phase** | | |
| | Crisaborole | 2.00 | 2.00 |
| | Diisopropyl adipate | 19.00 | 19.00 |
| | Diethyl sebacate | 19.00 | 19.00 |
| | Capric/caprylic triglycerides (Miglyol 810 ^{™}) | 9.50 | 9.50 |
| | Butylated hydroxyanisole | 0.10 | 0.10 |
| | Laureth 4 | 0.50 | 0.50 |
| | | | |
| | **Aqueous phase** | | |
| | Pemulen TR-2 | 0.50 | 0.50 |
| | Sodium citrate dehydrate | 0.123 | 0.123 |
| | Citric acid anhydrous | 0.071 | 0.071 |
| | Phenoxyethanol | 0.70 | 0.70 |
| | Natrosol 250L | 0.20 | 0.20 |
| | Sodium hydroxide 20% aqueous | 0.20 | 0.20 |
| | Water | 46.11 | 46.11 |
| | | | |
| Adjust (to pH6) | Sodium hydroxide (20% aqueous solution) | q.s. | q.s. |
| | Water | q.s. | q.s. |
| | | 100.00 | 100.00 |

The oil phase was made up with simple stirring. The aqueous phase was made by dispersing the pemulen, sodium citrate, citric acid and Phenoxyethanol in the water with moderate stirring. The sodium hydroxide was then added. The oil phase was then slowly added to the aqueous phase with stirring. The pH was then finally adjusted if necessary or made to quantity with water.

## Claims

1. A composition for topical application comprising a continuous aqueous phase, a discontinuous liquid oil phase and crisaborole,
wherein the discontinuous liquid oil phase is a liquid at 25 °C,
wherein the composition does not comprise a polyaphron dispersion,
wherein the discontinuous liquid oil phase comprises diisopropyl adipate and/or diethyl sebacate and/or dibutyl adipate, and
wherein the composition comprises less than 5 wt% by weight of the composition of glyceryl monostearate, cetyl alcohol, stearyl alcohol, stearic acid, cetyl ricinoleate, propylene glycol stearate, PEG-2 stearate, sorbitan monostearate, or mixtures of two or more thereof.

2. A composition according to claim 1, wherein the crisaborole is present in an amount of from 1 to 5 wt% by weight of the composition, preferably from 1.5 to 3.5 wt%.

3. A composition according to claim 1 or claim 2, wherein the crisaborole is predominantly in the discontinuous liquid oil phase.

4. A composition according to any of the preceding claims, wherein the composition comprises one or more further pharmaceutically active agents.

5. A composition according to any of the preceding claims, wherein the diisopropyl adipate and/or diethyl sebacate and/or dibutyl adipate are present in a total amount of at least 60 wt% by weight of the discontinuous liquid oil phase.

6. A composition according to any of the preceding claims, wherein the discontinuous liquid oil phase comprises, by weight of the composition:
(i) diisopropyl adipate in an amount of from 5 to 45 wt%;
(ii) diethyl sebacate and/or dibutyl adipate in a total amount of from 5 to 45 wt%; and
(iii) castor oil and/or caprylic/capric triglycerides in a total amount of from 5 to 45 wt%.

7. A composition according to any of the preceding claims having a total surfactant content of less than 5 wt% by weight of the composition, preferably less than 3 wt%.

8. A composition according to any of the preceding claims, wherein the composition comprises a surfactant comprising a crosslinked copolymer, the crosslinked copolymer comprising acrylic acid monomer units.

9. A composition according to any of the preceding claims, wherein the composition comprises at least 10 wt% water by weight of the composition; and/or
wherein the composition is in the form of a lotion or cream.

10. A composition according to any of the preceding claims, wherein at least 95 wt% of the crisaborole is dissolved in the composition, preferably throughout a temperature range of from 4 to 20 °C.

11. A composition according to any of the preceding claims, wherein the composition is chemically and/or physically stable for at least 12 months at 25 °C ± 2 °C, as measured at60% RH ± 5%; and/or wherein the composition is chemically and/or physically stable for at least 6 months at 40 °C ± 3 °C, as measured at 60% RH ± 5%.

12. A composition according to any of the preceding claims for use in the treatment of the human or animal body by therapy.

13. A composition according to any of claims 1 to 11 for use in the treatment of atopic dermatitis or psoriasis.

14. A package comprising from 20 to 300 g of the composition according to any of claims 1 to 11, or from 20 to 300 g of the composition for use according to claim 12 or claim 13.

## Patentansprüche

1. Zusammensetzung für eine topische Anwendung, die eine kontinuierliche wässrige Phase, eine diskontinuierliche flüssige Ölphase und Crisaborol umfasst,
wobei die diskontinuierliche flüssige Ölphase eine Flüssigkeit bei 25°C ist,
wobei die Zusammensetzung keine Poly-Aphron-Dispersion umfasst,
wobei die diskontinuierliche flüssige Ölphase Diisopropyladipat und/oder Diethylsebacat und/oder Dibutyladipat umfasst, und
wobei die Zusammensetzung weniger als 5 Gew.-% der Zusammensetzung Glycerolmonostearat, Cetylalkohol, Stearylalkohol, Stearinsäure, Cetyl Ricinoleat, Propylen-Glykolstearat, PEG-2 Stearat, Sorbitanmonostearat, oder Gemische aus zwei oder mehr davon umfasst.

2. Zusammensetzung nach Anspruch 1, wobei das Crisaborol in einer Menge von 1 bis 5 Gew.-% in der Zusammensetzung vorliegt, vorzugsweise in einer Menge von 1,5 bis 3,5 Gew.-%.

3. Zusammensetzung nach Anspruch 1 oder 2, wobei das Crisaborol überwiegend in der diskontinuierlichen flüssigen Ölphase vorliegt.

4. Zusammensetzung nach einem der vorhergehenden Ansprüche, wobei die Zusammensetzung einen oder mehr pharmazeutische Wirkstoffe umfasst.

5. Zusammensetzung nach einem der vorhergehenden Ansprüche, wobei das Diisopropyladipat und/oder Diethylsebacat und/oder Dibutyladipat in einer Gesamtmenge von mindestens 60 Gew.-% in der diskontinuierlichen flüssigen Ölphase vorliegen.

6. Zusammensetzung nach einem der vorhergehenden Ansprüche, wobei die diskontinuierliche flüssige Ölphase die Stoffe nach folgendem Gewicht der Zusammensetzung umfasst:
(i) Diisopropyladipat in einer Menge von 5 bis 45 Gew.-%;
(ii) Diethylsebacat und/oder Dibutyladipat in einer Gesamtmenge von 5 bis 45 Gew.-%; und
(iii) Rizinusöl und/oder Caprylic/Capric Triglyceride in einer Gesamtmenge von 5 bis 45 Gew.-%.

7. Zusammensetzung nach einem der vorhergehenden Ansprüche mit einem Gesamt-Tensidgehalt von weniger als 5 Gew.-% der Zusammensetzung, vorzugsweise weniger als 3 Gew.-%.

8. Zusammensetzung nach einem der vorhergehenden Ansprüche, wobei die Zusammensetzung ein Tensid mit einem vernetzten Copolymer umfasst, wobei das vernetzte Copolymer Acrylsäure-Monomereinheiten umfasst.

9. Zusammensetzung nach einem der vorhergehenden Ansprüche, wobei die Zusammensetzung mindestens 10 Gew.-% Wasser der Zusammensetzung umfasst, und/oder
wobei die Zusammensetzung in der Form einer Lotion oder Creme vorliegt.

10. Zusammensetzung nach einem der vorhergehenden Ansprüche, wobei zumindest 95 Gew.-% des Crisabols in der Zusammensetzung gelöst ist, vorzugsweise durchwegs bei einem Temperaturbereich von 4 bis 20°C.

11. Zusammensetzung nach einem der vorhergehenden Ansprüche, wobei die Zusammensetzung mindestens 12 Monate bei 25°C ± 2°C chemisch und/oder physikalisch stabil ist, gemessen bei 60% RH ± 5%; und/oder wobei die Zusammensetzung mindestens 6 Monate bei 40°C ± 3°C chemisch und/oder physikalisch stabil ist, gemessen bei 60% RH ± 5%.

12. Zusammensetzung nach einem der vorhergehenden Ansprüche zur Verwendung bei der therapeutischen Behandlung des menschlichen oder tierischen Körpers.

13. Zusammensetzung nach einem der Ansprüche 1 bis 11 zur Verwendung bei der Behandlung von atopischer Dermatitis und Psoriasis.

14. Packung, die von 20 bis 300 g der Zusammensetzung nach einem der Ansprüche 1 bis 11 umfasst, oder von 20 bis 300 g der Zusammensetzung zur Verwendung nach Anspruch 12 oder Anspruch 13.

## Revendications

1. Composition pour application topique comprenant une phase aqueuse continue, une phase huileuse liquide discontinue et du crisaborole,
dans laquelle la phase huileuse liquide discontinue est un liquide à 25°C,
dans laquelle la composition ne comprend pas de dispersion de polyaphron,
dans laquelle la phase huileuse liquide discontinue comprend de l'adipate de diisopropyle et/ou du sébacate de diéthyle et/ou de l'adipate de dibutyle, et
dans laquelle la composition comprend moins de 5 % en poids de la composition de monostéarate de glycéryle, d'alcool cétylique, d'alcool stéarylique, d'acide stéarique, de ricinoléate de cétyle, de stéarate de propylène glycol, de stéarate de PEG-2, de monostéarate de sorbitane, ou de mélanges de deux de ceux-ci ou plus.

2. Composition selon la revendication 1, dans laquelle le crisaborole est présent en une quantité de 1 à 5 % en poids de la composition, de préférence de 1,5 à 3,5 % en poids.

3. Composition selon la revendication 1 ou la revendication 2, dans laquelle le crisaborole est principalement dans la phase huileuse liquide discontinue.

4. Composition selon l'une quelconque des revendications précédentes, dans laquelle la composition comprend un ou plusieurs agents pharmaceutiquement actifs supplémentaires.

5. Composition selon l'une quelconque des revendications précédentes, dans laquelle l'adipate de diisopropyle et/ou le sébacate de diéthyle et/ou l'adipate de dibutyle sont présents en une quantité totale d'au moins 60 % en poids de la phase huileuse liquide discontinue.

6. Composition selon l'une quelconque des revendications précédentes, dans laquelle la phase huileuse liquide discontinue comprend, en poids de la composition :
(i) de l'adipate de diisopropyle en une quantité de 5 à 45 % en poids ;
(ii) du sébacate de diéthyle et/ou de l'adipate de dibutyle en une quantité totale de 5 à 45 % en poids ; et
(iii) de l'huile de ricin et/ou des triglycérides capryliques/capriques en une quantité totale de 5 à 45 % en poids.

7. Composition selon l'une quelconque des revendications précédentes, présentant une teneur totale en tensioactif inférieure à 5 % en poids de la composition, de préférence inférieure à 3 % en poids.

8. Composition selon l'une quelconque des revendications précédentes, dans laquelle la composition comprend un tensioactif comprenant un copolymère réticulé, le copolymère réticulé comprenant des unités monomères d'acide acrylique.

9. Composition selon l'une quelconque des revendications précédentes, dans laquelle la composition comprend au moins 10 % en poids d'eau par rapport au poids de la composition ; et/ou
dans laquelle la composition est sous la forme d'une lotion ou d'une crème.

10. Composition selon l'une quelconque des revendications précédentes, dans laquelle au moins 95 % en poids du crisaborole sont dissous dans la composition, de préférence dans une plage de températures allant de 4 à 20°C.

11. Composition selon l'une quelconque des revendications précédentes, dans laquelle la composition est chimiquement et/ou physiquement stable pendant au moins 12 mois à 25°C ± 2°C, après mesure à une humidité relative de 60 % ± 5 % ; et/ou dans laquelle la composition est chimiquement et/ou physiquement stable pendant au moins 6 mois à 40°C ± 3°C, après mesure à une humidité relative de 60 % ± 5 %.

12. Composition selon l'une quelconque des revendications précédentes pour utilisation dans le traitement du corps humain ou animal par thérapie.

13. Composition pharmaceutique selon l'une quelconque des revendications 1 à 11, pour utilisation dans le traitement de la dermatite atopique ou du psoriasis.

14. Conditionnement comprenant de 20 à 300 g de la composition selon l'une quelconque des revendications 1 à 11, ou de 20 à 300 g de la composition pour utilisation selon la revendication 12 ou la revendication 13.
